# EUROPÄISCHE PATENTSCHRIFT

(11) **EP 0 761 640 B1**
(45) Veröffentlichungstag und Bekanntmachung des Hinweises auf die Patenterteilung: **01.12.1999**
(21) Anmeldenummer: 96113730.4
(22) Anmeldetag: 28.08.1996
(51) Int. Cl.: C07C 69/712, C07C 67/08

(54) **Verfahren zur Herstellung von 2-(4-Hydroxyphenoxy)-propionsäureestern**
Process for the preparation of esters of 2-(4-hydroxyphenoxy)-propionic acid
Procédé de préparation d'esters de l'acide 2-(4-hydroxyphénoxy)-propionique

(30) Priorität: 06.09.1995 DE 19532815
(43) Veröffentlichungstag der Anmeldung: 12.03.1997
(73) Patentinhaber: BASF AKTIENGESELLSCHAFT, 67056 Ludwigshafen (DE)
(72) Erfinder: Wettling, Thomas, 67117 Limburgerhof (DE); Dimmler, Manfred, 67125 Dannstadt-Schauernheim (DE); Hupfeld, Bernd, 67346 Speyer (DE); Henkelmann, Jochem, 68165 Mannheim (DE)

(56) Entgegenhaltungen:
- EP-A- 0 259 995

## Beschreibung

Die vorliegende Erfindung betrifft ein Verfahren zur Herstellung von 2-(4-Hydroxyphenoxy)-propionsäureestern, insbesondere von 2-(4-Hydroxyphenoxy)-propionsäuremethylester hoher Reinheit durch mehrstufige Umsetzung des Veresterungsansatzes der entsprechenden 2-(4-Hydroxyphenoxy)-propionsäuren mit einem Alkohol in Anwesenheit einer destillierbaren Säure ausgewählt aus Salzsäure, Bromwasserstoffsäure, Chlorwasserstoff, Bromwasserstoff, halogenierten Essigsäurederivaten, Trifluormethansulfonsäure, Bortrifluorid und Verbindungen, die in wäßrigem Medium die vorstehend genannten destillierbaren Säuren freisetzen, ohne zusätzliche Reinigungsoperation.

Die Herstellung von Carbonsäureestern durch Umsetzung einer Carbonsäure mit einem Alkohol in Anwesenheit von katalytischen Mengen einer Säure ist z.B. aus Houben-Weyl, Methoden der Organischen Chemie, Band 5E, 1985, Seite 658 bis 663) bekannt. Typische Ausbeuten liegen dabei im Bereich von 72 bis 96 %. Um Ester hoher Reinheit zu erhalten ist es daher unerläßlich zusätzliche Reinigungsoperationen, wie z.B. eine Destillation, durchzuführen.

Aus dem Journal of Organic Chemistry 24, 261 bis 262 (1959) und Journal of Organic Chemistry 28, 2898 (1963) ist die Herstellung von Methylestern mit stöchiometrischen Mengen von Acetondimethylacetat als wasserbindendes Mittel bekannt. Nachteilig an diesem Verfahren ist der nicht quantitative Umsatz, die Entstehung von stöchiometrischen Mengen an Aceton und die aufwendige Herstellung des Acetondimethylacetats.

In Chem. Ind. Seiten 1568 bis 1569 (1968) wird für die Herstellung von Carbonsäuremethylestern Schwefelsäure als Katalysator in einer Soxleth-Apparatur empfohlen. Dabei verbleibt entweder der Katalysator im entstehenden Produkt, oder aber das Produkt muß z.B. durch Destillation des Esters von der Schwefelsäure befreit werden.

Zur Abtrennung des entstehenden Reaktionswassers wird die Verwendung eines Cosolvens, z.B. von Toluol empfohlen (Journal of Organic Chemistry 23, 108 bis 110 (1958)). Das Toluol wird dabei als Lösungsmittel für die Phasentrennung von der zurückbleibenden Schwefelsäure genutzt. Nachteilig daran ist die Verwendung großer Mengen an Schwefelsäure und eines zusätzlichen Lösungsmittels, das entweder gereinigt und rückgeführt, oder entsorgt werden muß. Erhebliche Probleme bereiten des weiteren im Produkt verbliebene Spuren des Säurekatalysators, bzw. von Umsetzungsprodukten der Carbonsäure mit dem Katalysator.

Bei Verwendung großer Mengen Salzsäure (bzw. von Chlorwasserstoff) als Säurekatalysator können bereits unter normalen Reaktionsbedingungen unerwünschte chlorierte Umsetzungsprodukte der eingesetzten Alkohole und Carbonsäuren entstehen (Houben-Weyl, Methoden der Organischen Chemie, Band 5/3, Seiten 831 bis 837 (1962)).

Der vorliegenden Erfindung lag daher die Aufgabe zugrunde, den zuvor genannten Nachteilen abzuhelfen, insbesondere ein Verfahren zu entwickeln um Hydroxyphenoxypropionsäureester hoher Reinheit herzustellen, ohne weitere Reinigungsoperationen und damit unter Verminderung von Reinigungsverlusten.

Demgemäß wurde ein neues und verbessertes Verfahren zur Herstellung von 2-(4-Hydroxyphenoxy)-propionsäureestern der allgemeinen Formel I 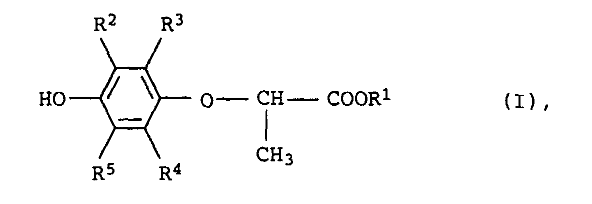 in der
- R¹: C₁- bis C₁₈-Alkyl, C₂- bis C₈-Alkinyl, Tetrahydrofurfuryl oder C₃- bis C₁₈-Alkoxyalkyl und
- R²,R³,R⁴,R⁵: Wasserstoff, C₁- bis C₄-Alkyl, Fluor, Chlor oder Brom
bedeuten, durch Umsetzung von 2-(4-Hydroxyphenoxy)-propionsäuren der allgemeinen Formel II 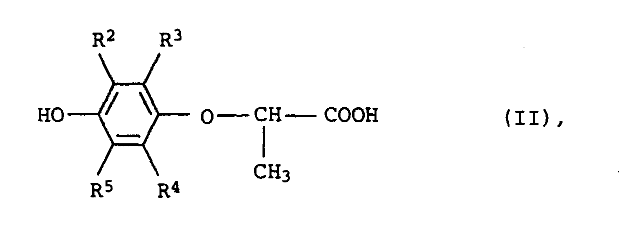 in der R², R³, R⁴ und R⁵ die oben genannten Bedeutungen haben, mit Alkoholen der allgemeinen Formel III R¹-OH (III), in der R¹ die oben genannten Bedeutungen hat, gefunden, welches dadurch gekennzeichnet ist, daß man die Veresterung der 2-Hydroxyphenoxy)-propionsäuren II bei Temperaturen von 20 bis 150°C und einem Druck von 0,05 bis 5 bar zwei- bis zehnmal mit einem Alkohol III in Gegenwart einer destillierbaren Säure ausgewählt aus Salzsäure, Bromwasserstoffsäure, Chlorwasserstoff, Bromwasserstoff, halogenierten Essigsäurederivaten, Trifluormethansulfonsäure, Bortrifluorid und Verbindungen, die in wäßrigem Medium die vorstehend genannten destillierbaren Säuren freisetzen, durchführt.

Das erfindungsgemäße Verfahren kann wie folgt durchgeführt werden:

Die erfindungsgemäße Umsetzung kann sowohl kontinuierlich als auch diskontinuierlich bei Temperaturen von 20 bis 150°C, bevorzugt 40 bis 130°C, besonders bevorzugt 50 bis 120°C und einem Druck von 0,05 bis 5 bar, bevorzugt 0,5 bis 2 bar, besonders bevorzugt Normaldruck (Atmosphärendruck) in der Regel durch Zumischen einer destillierbaren Säure zu einer Mischung aus 2-(4-Hydroxyphenoxy)-propionsäuren II und einem Alkohol III (eines frischen oder aus einer der Reaktionssequenzen zurückgewonnenen Alkohols) und anschließendes Entfernen des Alkohols und gegebenenfalls anderer flüchtiger Komponenten im Vakuum bei Temperaturen von 30 bis 100°C, bevorzugt 50 bis 90°C und einem Druck von 15 bis 720 mbar, bevorzugt 20 bis 700 mbar, besonders bevorzugt 30 bis 650 mbar, ohne Reinigung des Sumpfproduktes durchgeführt werden. Das Sumpfprodukt wird erfindungsgemäß ohne Reinigung noch weitere ein- bis neunmal (insgesamt also zwei- bis zehnmal), bevorzugt ein- bis viermal (insgesamt also zwei- bis fünfmal), besonders bevorzugt ein- bis zweimal (insgesamt also zwei-bis dreimal) bei Temperaturen von 20 bis 150°C, bevorzugt 40 bis 130°C, besonders bevorzugt 50 bis 120°C und einem Druck von 0,05 bis 5 bar, bevorzugt 0,5 bis 2 bar, besonders bevorzugt Normaldruck (Atmosphärendruck) in der Regel durch Zumischen einer destillierbaren Säure zu einer Mischung aus 2-(4-Hydroxyphenoxy)propionsäuren II und einem Alkohol III und anschließendes Entfernen des Alkohols und gegebenenfalls anderer flüchtiger Komponenten im Vakuum bei Temperaturen von 30 bis 100°C, bevorzugt 50 bis 90°C und einem Druck von 15 bis 720 mbar, bevorzugt 20 bis 700 mbar, besonders bevorzugt 30 bis 650 mbar, ohne Reinigung des Sumpfproduktes umgesetzt.

Das Molverhältnis vom Alkohol III zur 2-(4-Hydroxyphenoxy)propionsäure II beträgt in der Regel 1:1 bis 30:1, bevorzugt 1,5:1 bis 20:1, besonders bevorzugt 2:1 bis 15:1.

Das Molverhältnis von destillierbarer Säure zum Alkohol III beträgt in der Regel 0,0001:1 bis 0,1:1, bevorzugt 0,001:1 bis 0,05:1, besonders bevorzugt 0,005:1 bis 0,03:1.

Als destillierbare Säuren werden Salzsäure, Bromwasserstoffsäure, Chlorwasserstoff, Bromwasserstoff und halogenierte Essigsäurederivate, wie z.B. Trichloressigsäure, Trifluoressigsäure, Trifluormethansulfonsäure und Bortrifluorid, sowie des weiteren Verbindungen, die in wäßrigem Medium solche destillierbaren Säuren freisetzen (z.B. Methylchlorformiat + H₂O → Methanol, CO₂, Chlorwasserstoff), bevorzugt Salzsäure, Chlorwasserstoff und Bortrifluorid, besonders bevorzugt Salzsäure und Chlorwasserstoff, eingesetzt.

Das erfindungsgemäße Verfahren ist sowohl für die Veresterung von racemischen 2-(4-Hydroxyphenoxy)-propionsäuren, als auch für die chiralen Enantiomere, bevorzugt solche mit einem Enantiomerenverhältnis von R:S von kleiner als 5:95, besonders bevorzugt kleiner als 2:98, oder von größer als 95:5, besonders bevorzugt größer als 98:2, geeignet. Bei der Veresterung der chiralen Carbonsäure bleibt die Chiralität im entstandenen Ester im Wesentlichen (bevorzugt ± 5, besonders bevorzugt ± 2) erhalten.

In einer besonderen Ausführungsform wird die 2-(4-Hydroxyphenoxy)-propionsäure im entsprechenden Alkohol gelöst und für die erste Veresterungsstufe mit katalytischen Mengen einer Säure versetzt und erwärmt. Im Anschluß daran beginnt die Entfernung des Lösungsmittels im Vakuum der ersten Veresterungsstufe. Dabei wird überschüssiger Alkohol gemeinsam mit entstandenem Reaktionswasser, bzw. bereits in der Säure enthaltenem Wasser, möglichst rasch und bei Verwendung von Methanol als Alkoholkomponente bevorzugt unter vermindertem Druck um den Wasseranteil im Destillat zu erhöhen, abdestilliert. Dabei wird gleichzeitig ein großer Teil der zuvor zugesetzten Säure mit entfernt. Die Temperatur der Produktschmelze wird so eingestellt, daß das Produkt gerade noch flüssig und gut rührbar ist.

Anschließend beginnt die zweite Veresterungsstufe mit dem Zudosieren von frischem Alkohol und einer bevorzugt verringerten Menge der Säure. Falls zur Erreichung des erwünschten Umsatzes zwei Veresterungsstufen ausreichen, wird bevorzugt eine der ersten Stufe vergleichbare Alkoholmenge eingesetzt. Falls drei oder mehr Veresterungsstufen zur Erreichung des gewünschten Umsatzgrades erforderlich sind, so kann bei der zweiten und allen folgenden Veresterungsstufen außer der letzten Stufe die dafür eingesetzte Alkoholmenge in vorteilhafter Weise deutlich reduziert werden (z.B. um ein Drittel). Die zweite und alle folgenden Destillationsphasen werden in Analogie zur Ersten durchgeführt. Nach der letzten erforderlichen Alkoholdestillation (Alkoholdestillat wird für die erste Veresterungsstufe der folgenden Charge aufbewahrt) wird die Temperatur des Reaktionsgemisches soweit abgesenkt, daß die Schmelze gerade noch gut rührbar ist (bevorzugt 60 bis 90°C) und zur Entfernung von Spuren der eingesetzten Säure ein Unterdruck an das Reaktionsgefäß angelegt. Zur Beschleunigung der Entfernung leicht flüchtiger Verunreinigungen empfiehlt sich das gleichzeitige Einleiten eines Stickstoffstromes in das Reaktionsmedium.

Der nach dem erfindungsgemäßen Verfahren hergestellte 2-(4-Hydroxyphenoxy)-propionsäureester zeichnet sich, ohne die Notwendigkeit einer Reinigungsstufe, durch eine Reinheit von >99 %, einen Wassergehalt von <0,1 %, einen Halogenidgehalt von <100 ppm, einen organischen Halogengehalt von <50 ppm und einen Gehalt an weiteren verunreinigenden Einzelverbindungen, insbesondere Hydroxyphenoxy-propionsäureoligomere von <0,1 % aus.

Abschließend kann das Produkt ohne weitere Reinigungsoperationen für Folgeumsetzungen direkt eingesetzt, oder für die Lagerung und den Transport konfektioniert (z.B. geschuppt) werden.

Die Substituenten R¹, R², R³, R⁴ und R⁵ in den Verbindungen I, II und III haben folgende Bedeutungen:
- R¹: - C₁- bis C₁₈-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, tert-Butyl, Ethylhexyl,
- C₃- bis C₁₈-Alkoxyalkyl, bevorzugt C₃- bis C₁₂-Alkoxyalkyl, bevorzugt C₃- bis C₈-Alkoxyalkyl wie Methoxyethyl und Ethoxyethyl,
- C₂- bis C₈-Alkinyl, bevorzugt Propargyl,
- Tetrahydrofurfuryl,
- R²,R³,R⁴,R⁵: - Wasserstoff,
- C₁- bis C₄-Alkyl wie Methyl, Ethyl, n-Propyl, iso-Propyl, n-Butyl, iso-Butyl und tert-Butyl, bevorzugt Methyl und Ethyl, besonders bevorzugt Methyl,
- Fluor, Chlor oder Brom.

Insbesonders sind die Verbindungen I, II und III bevorzugt, in denen R¹ für Methyl und R², R³, R⁴ und R⁵ jeweils für Wasserstoff stehen.

Die 2-(4-Hydroxyphenoxy)-propionsäureester I eignen sich als Vorprodukte in der Pflanzenschutz- und Arzneimittelsynthese.

### Beispiele

### Beispiel 1

Zu 191 g (1 mol) 2-(4-Hydroxyphenoxy)-propionsäure, feucht (5 % H₂O-Gehalt) und 160 g (5 mol) Methanol wurden 2 g (0,02 mol) 36 %ige Salzsäure gegeben und 2 h unter Rückfluß erhitzt. Nach Entfernung des Lösungsmittels i.Vak. (600 bis 30 mbar/80°C Sumpftemperatur) erhielt man 194 g Hydroxyphenoxypropionsäuremethylester (Estergehalt: 95,9 %, Chloridwert: 2100 ppm).

Das zuvor erhaltene Produkt (Chloridwert: 2100 ppm) wurde erneut mit 160 g (5 mol) Methanol und 2 g (0,02 mol) 36 %iger Salzsäure 1,5 h unter Rückfluß erhitzt. Nach analoger Aufarbeitung erhielt man 196 g Hydroxyphenoxypropionsäuremethylester (Estergehalt: 99,4 %, Chloridwert: 190 ppm).

Das zuvor erhaltene Produkt (Chloridwert: 190 ppm) wurde erneut mit 160 g (5 mol) Methanol und 2 g (0,02 mol) 36 %iger Salzsäure 1 h unter Rückfluß erhitzt. Nach analoger Aufarbeitung erhielt man 196 g (100 %) [Gehalt: 99,7 %] Hydroxyphenoxypropionsäuremethylester (Chloridwert: 29 ppm; organisches Chlor: < 10 ppm, andere Verunreinigungen: < 0,1 %, [Hydroxyphenoxypropionsäure nicht nachweisbar]).

### Beispiel 2

Analog Beispiel 1 wurde 191 g (1 mol) R-2-(4-Hydroxyphenoxy)propionsäure, feucht (5 % H₂O-Gehalt) mit einem R:S-Verhältnis von 99,3:0,7 umgesetzt. Nach drei Veresterungsstufen erhielt man 196 g (100 %) [Gehalt: 99,7 %] R-2-(4-Hydroxyphenoxy)-propionsäuremethylester mit einem Enantiomerenverhältnis R:S von 99,4:0,6 (Chloridwert: 29 ppm; organisches Chlor: < 10 ppm, andere Verunreinigungen: < 0,1 %, [R-Hydroxyphenoxypropionsäure nicht nachweisbar]).

### Beispiel 3

In eine Mischung aus 191 g (1 mol) 2-(4-Hydroxyphenoxy)-propionsäure, feucht (5 % H₂O-Gehalt) und 160 g (5 mol) Methanol wurde 0,73 g (0,02 mol) Chlorwasserstoff eingegast und analog Beispiel 1 umgesetzt und aufgearbeitet. Man erhielt 196 g (100 %) [Gehalt: 99,7 %] 2-(4-Hydroxyphenoxy)-propionsäuremethylester (Chloridwert: 34 ppm; organisches Chlor: < 10 ppm, andere Verunreinigungen: < 0,1 %, [Hydroxyphenoxypropionsäure nicht nachweisbar]).

### Beispiel 4

Analog Beispiel 1 wurden in eine Mischung aus 182 g (1 mol) R-2-(4-Hydroxyphenoxy)-propionsäure (Enantiomerenverhältnis R:S von 99,2:0,8)und 160 g (5 mol) Methanol 0,5 g (0,013 mol) Chlorwasserstoff eingegast und 4 h unter Rückfluß erhitzt. Nach Entfernung des Lösungsmittels i.Vak. wurden erneut 160 g Methanol ohne weiteren Chlorwasserstoff zugegeben und 4 h unter Rückfluß erhitzt und das Lösungsmittel i.Vak. entfernt. Anschließend wurde Stickstoff bei einem Unterdruck von 15 mbar 5 h durch den Reaktionssumpf geleitet. Man erhielt 196 g (100 %) [Gehalt: 99,6 %] R-2-(4-Hydroxyphenoxy)-propionsäuremethylester mit einem Enantiomerenverhältnis R:S von 99,2:0,8 (Chloridwert: 33 ppm; organisches Chlor nicht nachweisbar, andere Verunreinigungen: < 0,1 %, [R-Hydroxyphenoxypropionsäure nicht nachweisbar]).

### Beispiel 5

Analog Beispiel 4 wurde jedoch in der ersten Veresterungsstufe 1 g (0,012 mol) Bromwasserstoff eingegast. Man erhielt 196 g (100 %) [Gehalt: 99,4 %] 2- (4-Hydroxyphenoxy)-propionsäuremethylester (0,3 % Hydroxyphenoxypropionsäure).

### Beispiel 6

Analog Beispiel 1 wurde in der ersten Veresterungsstufe 149 g Methanol aus einem Destillat der dritten Veresterungsstufe des Beispiels 1 eingesetzt. Man erhielt 196 g (100 %) [Gehalt: 99,7 %] 2-(4-Hydroxyphenoxy)-propionsäuremethylester (0,1 % Hydroxyphenoxypropionsäure).

### Beispiel 7

193 g (1 mol) R-2-(4-Hydroxyphenoxy)-propionsäure, feucht (6 % H₂O-Gehalt) und 160 g rückgeführtes Methanol wurden mit 1,5 g einer 36 %igen Salzsäure 2 h unter Rückfluß erhitzt. Nach Entfernung des Lösungsmittels i.Vak. (700 bis 20 mbar/90°C Sumpftemperatur) wurde bei 70°C 130 g frisches Methanol zudosiert, 0,3 g (0,008 mol) Chlorwasserstoff eingegast, 2 h unter Rückfluß erhitzt und das Lösungsmittel wie zuvor i.Vak. entfernt.

Anschließend wurde bei 70°C 160 g frisches Methanol zudosiert, 4 h unter Rückfluß erhitzt und das Lösungsmittel wie zuvor i.Vak. entfernt. Danach wurde bei vermindertem Druck (ca. 500 mbar) Stickstoff durch den Reaktionssumpf geleitet. Man erhielt 196 g (100 %) [Gehalt: 99,7 %] R-2-(4-Hydroxyphenoxy)propionsäuremethylester mit einem Enantiomerenverhältnis R:S von 99,5:0,5 (Chloridwert: 37 ppm; organisches Chlor nicht nachweisbar, 0,1 % R-Hydroxyphenoxypropionsäure).

### Beispiele 8 bis 12

Analog Beispiel 6 wurden insgesamt fünf Chargen (Beispiele 8 bis 12) R-2-(4-Hydroxyphenoxy)-propionsäuremethylester hergestellt und dabei jeweils das zurückgewonnene Destillat der dritten Destillation des vorangegangenen Ansatzes für die erste Veresterungsstufe des folgenden Ansatzes eingesetzt. Die Ergebnisse sind in Tabelle 1 zusammengestellt.

**Tabelle 1**

| Beispiel Nr. | Hydroxyphenoxypropionsäuremethylester [Gehalt in %] | Hydroxyphenoxypropionsäure [Gehalt in %] | Chlorid [Gehalt in ppm] |
|---|---|---|---|
| 8 | 99,6 | 0,1 | 27 |
| 9 | 99,7 | 0,1 | 41 |
| 10 | 99,7 | 0,1 | 36 |
| 11 | 99,6 | 0,1 | 33 |
| 12 | 99,7 | 0,1 | 35 |

Organisches Chlor konnte jeweils nicht nachgewiesen werden (<10ppm). Andere Verunreinigungen, insbesondere Oligomere der Hydroxyphenoxypropionsäure lagen unter 0,1 % (je Einzelkomponente und < 0,3 % insgesamt).

### Beispiele 13 bis 16

Zu 365 g (2 mol) R-2-(4-Hydroxyphenoxy)-propionsäure und 300 g eines Alkohol der Tabelle 2 wurden 3 g Salzsäure gegeben, 1 h unter Rückfluß erhitzt und der überschüssige Alkohol unter Normaldruck abdestilliert. Anschließend wurden 300 g desselben Alkohols zugefügt und 0,7 g Chlorwasserstoff eingegast und 2 h unter Rückfluß erhitzt. Danach wurde das Lösungsmittel i.Vak. (65°C/20 mbar) entfernt und 2 h Stickstoff durch den Destillationssumpf geleitet. Die Ergebnisse sind in Tabelle 2 zusammengestellt.

**Tabelle 2**

| Beispiel Nr. | Alkohol | R-Hydroxyphenoxypropionsäure-......-ester | Gehalt [%] |
|---|---|---|---|
| 13 | Ethanol | -ethyl- | 99,4 |
| 14 | n-Butanol | -n-butyl- | 99,5 |
| 15 | iso-Butanol | -iso-butyl- | 99,5 |
| 16 | Ethoxyethanol | -ethoxyethyl- | 99,4 |

## Patentansprüche

1. Verfahren zur Herstellung von 2-(4-Hydroxyphenoxy)-propionsäureestern der allgemeinen Formel I 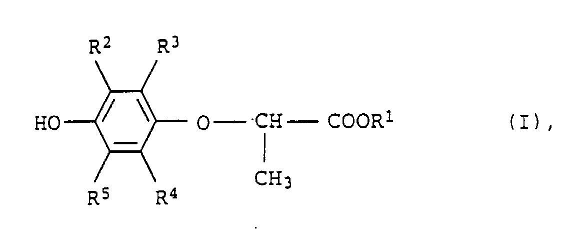 in der
R¹ C₁- bis C₁₈-Alkyl, C₂- bis C₈-Alkinyl, Tetrahydrofurfuryl oder C₃- bis C₁₈-Alkoxyalkyl und
R²,R³,R⁴,R⁵ Wasserstoff, C₁- bis C₄-Alkyl, Fluor, Chlor oder Brom
bedeuten, durch Umsetzung von 2-(4-Hydroxyphenoxy)-propionsäuren der allgemeinen Formel II 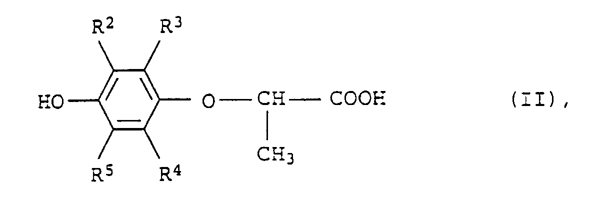 in der R², R³, R⁴ und R⁵ die oben genannten Bedeutungen haben, mit Alkoholen der allgemeinen Formel III R¹-OH (III), in der R¹ die oben genannten Bedeutungen hat, dadurch gekennzeichnet, daß man die Veresterung der 2-(4-Hydroxyphenoxy)-propionsäuren II bei Temperaturen von 20 bis 150°C und einem Druck von 0,05 bis 5 bar zwei- bis zehnmal mit einem Alkohol III in Gegenwart einer destillierbaren Säure ausgewählt aus Salzsäure, Bromwasserstoffsäure, Chlorwasserstoff, Bromwasserstoff, halogenierten Essigsäurederivaten, Trifluormethansulfonsäure, Bortrifluorid und Verbindungen, die in wäßrigem Medium die vorstehend gennanten destillierbaren Säuren freisetzen, durchführt.

2. Verfahren zur Herstellung von 2-(4-Hydroxyphenoxy)-propionsäureestern I nach Anspruch 1, dadurch gekennzeichnet, daß man den überschüssigen Alkohol III im Vakuum entfernt.

3. Verfahren zur Herstellung von 2-(4-Hydroxyphenoxy)-propionsäureestern I nach den Ansprüchen 1 bis 2, dadurch gekennzeichnet, daß man die 2-(4-Hydroxyphenoxy)-propionsäureester I nicht reinigt.

4. Verfahren zur Herstellung von 2-(4-Hydroxyphenoxy)-propionsäureestern I nach den Ansprüchen 1 bis 3, dadurch gekennzeichnet, daß man die 2-(4-Hydroxyphenoxy)-propionsäure II zwei- bis fünfmal mit einem Alkohol III umsetzt.

5. Verfahren zur Herstellung von 2-(4-Hydroxyphenoxy)-propionsäureestern I nach den Ansprüchen 1 bis 4, dadurch gekennzeichnet, daß man den Alkohol III zur 2-(4-Hydroxyphenoxy)propionsäure II im Molverhältnis von 1:1 bis 30:1 einsetzt.

6. Verfahren zur Herstellung von 2-(4-Hydroxyphenoxyl-propionsäureestern I nach den Ansprüchen 1 bis 5, dadurch gekennzeichnet, daß man die destillierbare Säure zum Alkohol III im Molverhältnis von 0,0001:1 bis 0,1:1 einsetzt.

7. Verfahren zur Herstellung von 2-(4-Hydroxyphenoxy)-propionsäureestern I nach den Ansprüchen 1 bis 6, dadurch gekennzeichnet, daß der Substituent R¹ Methyl, Ethyl, n-Propyl, iso-Propyl, n-Butyl, iso-Butyl, tert-Butyl, Ethylhexyl, Methoxyethyl, Propargyl-, Tetrahydrofurfuryl- und Ethoxyethyl bedeutet.

8. Verfahren zur Herstellung von 2- (4-HYdroxyphenoxy)-propionsäureestern I nach den Ansprüchen 1 bis 7, dadurch gekennzeichnet, daß der Substituent R¹ Methyl bedeutet.

9. Verfahren zur Herstellung von 2-(4-Hydroxyphenoxy)-propionsäureestern I nach den Ansprüchen 1 bis 8, dadurch gekennzeichnet, daß die Substituenten R², R³, R⁴ und R⁵ wasserstoff bedeuten.

10. Verfahren zur Herstellung von 2-(4-Hydroxyphenoxy)-propionsäureestern I nach den Ansprüchen 1 bis 9, dadurch gekennzeichnet, daß man 2-(4-Hydroxyphenoxy)-propionsäuren II mit einem Enantiomerenverhältnis R:S von kleiner als 5:95 oder größer als 95:5 einsetzt.

11. Verfahren zur Herstellung von 2-(4-Hydroxyphenoxy)-propionsäureestern I nach den Ansprüchen 1 bis 10, dadurch gekennzeichnet, daß man als destillierbare Säure Salzsäure oder Chlorwasserstoff einsetzt.

## Claims

1. A process for preparing 2-(4-hydroxyphenoxy)propionic esters of the formula I 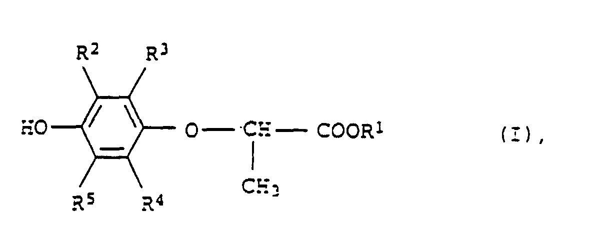 in which
R¹ is C₁- to C₁₈-alkyl, C₂- to C₈-alkynyl, tetrahydrofurfuryl or C₃- to C₁₈-alkoxyalkyl and
R²,R³,R⁴,R⁵ are hydrogen, C₁- to C₄-alkyl, fluorine, chlorine or bromine
by reacting 2-(4-hydroxyphenoxy)propionic acids of the formula II 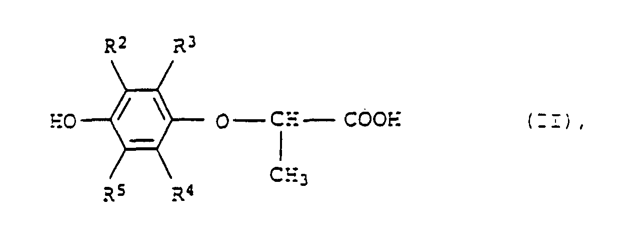 in which R², R³, R⁴ and R⁵ are as defined above with alcohols of the formula III R¹-H (III) in which R¹ is as defined above, which comprises carrying out the esterification of the 2-(4-hydroxyphenoxy)propionic acids II two to ten times at from 20 to 150°C and a pressure of from 0.05 to 5 bar with an alcohol III in the presence of a distillable acid selected from the group consisting of hydrochloric acid, hydrobromic acid, hydrogen chloride, hydrogen bromide, halogenated acetic acid derivatives, trifluoromethanesulfonic acid, boron trifluoride and compounds which, in aqueous medium, liberate the abovementioned distillable acids.

2. A process for preparing 2-(4-hydroxyphenoxy)propionic esters I as claimed in claim 1, wherein the excess alcohol III is removed under reduced pressure.

3. A process for preparing 2-(4-hydroxyphenoxy)propionic esters as claimed in claim 1 or 2, wherein the 2-(4-hydroxyphenoxy)propionic ester I is not purified.

4. A process for preparing 2-(4-hydroxyphenoxy)propionic esters I as claimed in any of claims 1 to 3, wherein the 2-(4-hydroxyphenoxy)propionic acid II is reacted two to five times with an alcohol III.

5. A process for preparing 2-(4-hydroxyphenoxy)propionic esters I as claimed in any of claims 1 to 4, wherein the alcohol III is employed in a molar ratio of from 1:1 to 30:1 relative to the 2-(4-hydroxyphenoxy)propionic acid II.

6. A process for preparing 2-(4-hydroxyphenoxy)propionic esters I as claimed in any of claims 1 to 5, wherein the distillable acid is employed in a molar ratio of from 0.0001:1 to 0.1:1 relative to the alcohol III.

7. A process for preparing 2-(4-hydroxyphenoxy)propionic esters I as claimed in any of claims 1 to 6, wherein the substituent R¹ is methyl, ethyl, n-propyl, isopropyl, n-butyl, isobutyl, tert-butyl, ethylhexyl, methoxyethyl, propargyl-, tetrahydrofurfuryl- and ethoxyethyl.

8. A process for preparing 2-(4-hydroxyphenoxy)propionic esters I as claimed in any of claims 1 to 7, wherein the substituent R¹ is methyl.

9. A process for preparing 2-(4-hydroxyphenoxy)propionic esters I as claimed in any of claims 1 to 8, wherein the substituents R², R³, R⁴ and R⁵ are hydrogen.

10. A process for preparing 2-(4-hydroxyphenoxy)propionic esters I as claimed in any of claims 1 to 9, wherein 2-(4-hydroxyphenoxy)propionic acids II having an enantiomer ratio R:S of less than 5:95 or more than 95:5 are employed.

11. A process for preparing 2-(4-hydroxyphenoxy)propionic esters I as claimed in any of claims 1 to 10, wherein the distillable acid used is hydrochloric acid or hydrogen chloride.

## Revendications

1. Procédé pour préparer des esters 2-(4-hydroxyphénoxy)propioniques de formule générale I 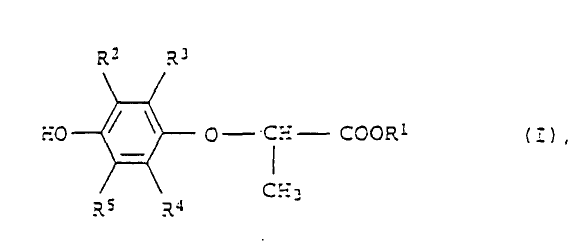 dans laquelle
R¹ représente un groupe alkyle en C1-C18, alcynyle en C2-C8, tétrahydrofurfuryle ou alcoxyalkyle en C3-C18 et
R², R³, R⁴, R⁵ représentent l'hydrogène, des groupes alkyle en C1-C4, le fluor, le chlore ou le brome,
par réaction des acides 2-(4-hydroxyphénoxy)propioniques de formule générale II 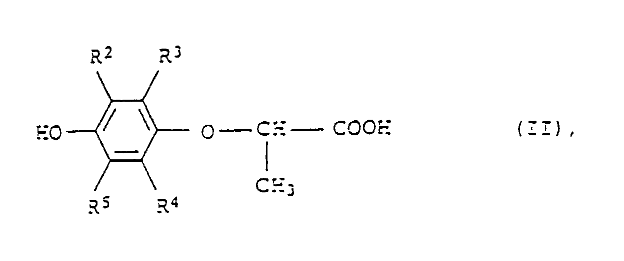 dans laquelle R², R³, R⁴ et R⁵ ont les significations indiquées ci-dessus, avec des alcools de formule générale III R¹-OH (III) dans laquelle R¹ a les significations indiquées ci-dessus, caractérisé par le fait que l'on procède à l'estérification des acides 2-(4-hydroxyphénoxy)propioniques II par un alcool III à deux à dix reprises à des températures de 20 à 150°C, sous une pression de 0,05 à 5 bar, en présence d'un acide distillable choisi parmi l'acide chlorhydrique, l'acide bromhydrique, le chlorure d'hydrogène, le bromure d'hydrogène, des dérivés halogénés de l'acide acétique, l'acide trifluorométhanesulfonique, le trifulorure de bore et les composés qui, en milieu aqueux, libèrent les acides distillables dont on vient de parler.

2. Procédé de préparation des esters 2-(4-hydroxyphénoxy)propioniques selon la revendication 1, caractérisé par le fait que l'on élimine l'excès d'alcool III sous vide.

3. Procédé de préparation des esters 2-(4-hydroxyphénoxy)propioniques I selon les revendications 1 à 2, caractérisé par le fait que l'ester 2-(4-hydroxyphénoxy)propionique I n'est pas purifié.

4. Procédé de préparation des esters 2-(4-hydroxyphénoxy)propioniques I selon les revendications 1 à 3, caractérisé par le fait que l'on fait réagir l'acide 2-(4-hydroxyphénoxy)propionique II à deux à cinq reprises avec un alcool III.

5. Procédé de préparation des esters 2-(4-hydroxyphénoxy)propioniques I selon les revendications 1 à 4, caractérisé par le fait que l'alcool III est mis en oeuvre à un rapport molaire de 1 : 1 à 30 : 1 avec l'acide 2-(4-hydroxyphénoxy)propionique II.

6. Procédé de préparation des esters 2-(4-hydroxyphénoxy)propioniques I selon les revendications 1 à 5, caractérisé par le fait que l'on utilise l'acide distillable dans un rapport molaire de 0,0001 : 1 à 0,1 : 1 avec l'alcool III.

7. Procédé de préparation des esters 2-(4-hydroxyphénoxy)propioniques I selon les revendications 1 à 6, caractérisé par le fait que R¹ représente un groupe méthyle, éthyle, n-propyle, isopropyle, n-butyle, isobutyle, tert-butyle, éthylhexyle, méthoxyéthyle, propargyle, tétrahydrofurfuryle ou éthoxyéthyle.

8. Procédé de préparation des esters 2-(4-hydroxyphénoxy)propioniques I selon les revendications 1 à 7, caractérisé par le fait que R¹ représente un groupe méthyle.

9. Procédé de préparation des esters 2-(4-hydroxyphénoxy)propioniques I selon les revendications 1 à 8, caractérisé par le fait ue R², R³, R⁴ et R⁵ représentent l'hydrogène.

10. Procédé de préparation des esters 2-(4-hydroxyphénoxy)propioniques I selon les revendications 1 à 9, caractérisé par le fait que l'on met les acides 2-(4-hydroxyphénoxy)propioniques II en oeuvre à un rapport inférieur à 5 : 95 ou supérieur à 95 : 5 entre les énantiomères R : S.

11. Procédé de préparation des esters 2-(4-hydroxyphénoxy)propioniques I selon les revendications 1 à 10, caractérisé par le fait que l'acide distillable utilisé est l'acide chlorhydrique ou le chlorure d'hydrogène.
